# EUROPEAN PATENT APPLICATION

(11) **EP 0 900 839 A1**
(43) Date of publication of application: **10.03.1999**
(21) Application number: 98113950.4
(22) Date of filing: 25.07.1998
(51) Int. Cl.: C12N 1/20

(54) **Culture medium for isolating and typing helicobacteria, in particular Helicobacter pylori**

(30) Priority: 25.07.1997 IT CA970024
(71) Applicant: Microbiol S.n.c., 09010 Uta (Cagliari) (IT)
(72) Inventor: SCHIVO, Maria Laura, Cagliari (IT); LOI, Giovanni, Cagliari (IT); SADDI, Barbara, Cagliari (IT); SERRA, Corrado, Cagliari (IT)
(74) Representative: Luppi, Luigi

(57) **Abstract**

A culture medium for isolating and typing helicobacteria, comprising an egg yolk suspension and its components.

## Description

The present invention refers to a new culture medium for selectively isolating and recognising the microorganisms normally responsible for clinical infections appertaining to the Helicobacter genus from tissue biopsies, saliva and stool specimens.

Helicobacteria (bacteria belonging to the genus Helicobacter) are Gram negative microorganisms of a curve or helicoidal shape varying in length and width (0,5 x 0,2-0,5 mm). They are asporogenous and mobile because of the presence of multiple sheathed flagella. During a stationary growth phase of a culture the small rod or coccoidal shape prevail. Among the helicobacteria that are involved in human pathology, the Helicobacter pylori is universally recognised as being the etiologic agent responsible for type B gastritis (active chronic antral gastritis).

Helicobacter pylori can be resistant to chemicoantibiotics agents commonly used in therapies aimed at eliminating this bacteria species, therefore it is necessary to introduce specific therapies aimed at overcoming this problem.

There are currently in commerce culture media for selective culture of Helicobacter pylori which is cultivated using culture media, the formulation of which derives from media normally used for Campylobacter (until 1989 Helicobacter pylori was denominated Campylobacter pylori), because it has many characteristics in common with campylobacteria. These media are mainly constituted by a base product enriched with sera or plasma or horse, bovine or human blood made selective by a mixture of antibiotics and antifungal agents. These media, whilst being selective, have little differentiating power, because they do not contain coloured substrates for recognising helicobacteria from those microorganisms that can anyway develop notwithstanding the selective qualities of the culture medium. Furthermore, the media currently available in commerce do not allow the development of the metabolically inactive forms (coccoidal forms) of Helicobacter pylori thus making the isolation often difficult, or even impossible, from biological material particularly contaminated such as saliva and stool, the subsequent identification of the microorganism by metabolic and/or biochemical tests and the execution of the antibiogram.

The considerations mentioned above, together make evident the importance and the need of media for rapidly isolating and recognising Helicobacter pylori in culture plates. Such a need is satisfied by the present invention. It makes available a new medium, semi-synthetic, selective, differential for isolating and recognising Helicobacter pylori in culture plates, whether the germ is found in the spiral form (metabolically active) or in the coccoidal form (metabolically non-active) the culture of which is normally considered very difficult. or even impossible, in traditional culture media. Furthermore, since the bacteria are maintained in the vegetative form, it is possible to prepare pure cultures and bacterial suspensions having an elevated level of live germs, which makes easy the execution of biochemical and metabolic tests necessary for identifying the germ and executing the antibiogram.

According to the present invention, there is provided a culture medium for helicobacteria, in particular for Helicobacter pylori, characterised in that it comprises a suspension of egg yolk and its components.

The suspension of egg yolk and its components constitutes the main enrichment factor of the culture medium, which allows the development of the Helicobacter pylori to be obtained in a short time, but above all allows the development of the metabolically inactive forms (coccoidal forms) and their reconversion to the spiral form phase, that tend to become easily inactive on traditional culture media, thus allowing the isolated breed to be maintained in a spiral form, metabolically active, for long periods of time.

It is advantageous to use a suspension of fresh egg yolk obtained from a grade A fresh chicken or duck's eggs (approximately 60 grams).

The culture medium according to the invention, can furthermore include one or more of the following components:
a) a nutritive base suitable for the growth of the helicobacteria.
b) a mixture of selective substances that inhibits fungi, Gram positive and Gram negative germs different from the helicobacteria.
c) a system for differentiating the helicobacteria from other microorganisms that may sometimes develop on the medium.
d) a solution of essential growth factors that constitute a further enrichment for the helicobacter pylori.
e) a gel substance.
f) distilled or de-ionized water.

It is convenient to choose as component a) a nutritive base that is capable of favouring an optimum growth of the helicobacteria in the shortest possible time and is simple to be prepared. The commercial Columbia Broth, amongst the known nutritive bases, is preferred.

A mixture constituted by numerous antifungal substances and antibiotics which, according to current literature, the Helicobacter pylori resists, may be used as component b). For this purpose, the antibiotics and antifungal substances preferred are: Nistatin, Amphoterycin B, Vancomycin, Colystin, Trimethoprim lactate, Cefsulodin.

With reference to component c), the differentiation of the helicobacteria is obtainable by incorporating Tetrazolium chloride in the culture medium, which may be used (reduced) by the microorganisms and leads to colonies of a red colour. Such a colour, in conjunction with the morphology of the colonies allows the Helicobacter pylori colonies to be differentiated from other microorganisms that may sometimes develop in the culture medium.

With reference to component d), a solution of substances is used, which are normally considered growth factors more or less essential for an optimum development of the microorganisms. The mixture may be constituted by dextrose, vitamin B12, L-glutamine, Cysteine Hcl, L-cystine, Thiamine Hcl, Guanine Hcl, Adenine SO4, P-amino benzoic acid, Cocarboxylase and ferric nitrate.

Finally, the medium is made solid by adding a gel substance such as agar (component e) to the base medium that is dissolved in distilled or deionised water during the preparation.

The solid medium is opaque and yellow; when the helicobacteria that have the possibility of metabolising the Tetrazolium grow on it, the colonies become red coloured with golden reflections on the surface.

According to a preferred version of the present invention, the culture medium comprises (the concentrations refer to 1000 ml of final medium):
- Columbia broth medium from 5 to 80 g, 42,5 g being particularly preferred;
- a mixture of antifungal substances and chemicoantibiotics the composition of which is shown in the following schedule: Schedule 1. Composition, range of concentration and recommended concentrations for the mixture of antifungal substances and antibiotics.

| Antifungal Chemicoantibiotic | Range of concentration (mg/l) | Concentrations recommended (mg/l) |
|---|---|---|
| Vancomycin | 3,00 - 15,00 | 9,8 |
| Trimethoprim lactate | 1,50 - 10,00 | 4,8 |
| Cefsulodin | 1,50 - 10,00 | 4,8 |
| Colystin | 2,00 - 20,00 | 7,3 |
| Amphoterycin B | 1,50 - 10,00 | 4,8 |
| Nistatin | 3000 - 20000(U.I./l) | 12.000(U.I./l) |

- a differentiating system constituted by Tetrazolium salts, preferably Tetrazolium chloride in a watery solution from 5,00 to 150 mg, 45,00 mg/l of final medium being particularly preferred.
- a suspension of chicken or duck's egg yolk from 2 ml to 200 ml; particularly preferred 30 ml of fresh yolk.
- solution of growth factors the composition of which is shown in schedule 2:

Schedule 2. Composition, range of concentrations and recommended concentrations for the mixture of growth factors.

| Substance recommended | Range of concentration (mg/l) | Concentration (mg/l) |
|---|---|---|
| Dextrose | 200,00 - 5000,00 | 2000 |
| Vitamin B12 | 0,02 - 5,00 | 0,2 |
| L-glutamine | 20,00 - 1000,00 | 200 |
| Cysteine Hcl | 20,00 - 500,00 | 120 |
| L-Cystine | 2,00 - 50,00 | 20 |
| Thiamine Hcl | 0,1 - 0,20 | 0,06 |
| Guanine Hcl | 0,05 - 1,00 | 0,6 |
| Adenine SO₄ | 1,00 - 50,00 | 20 |
| P-amino benzoic acid | 0,05 - 1,00 | 0,20 |
| Cocarboxylase | 0,023 - 10,00 | 2 |
| Ferric nitrate | 0,05 - 2,00 | 0,4 |

- agar from 10 to 20 g, 15g being particularly preferred;
- water 1 litre; the pH will be between 6,5 and 7,5.

The medium according to the invention can be prepared in the following way:
the basis of Columbia, agar and distilled water, are mixed together at a temperature of 95° - 100°C, and is then sterilised in an autoclave at a temperature of 121°C, at a relative pressure of 1 atmosphere for approximately 15 minutes. When the sterilisation is completed, the medium is brought to a temperature of 48°C and the mixture of antibiotics/antifungal agents, the growth factors solution, the solution of Tetrazolium and the egg yolk suspension are added. The mixture of antibiotics/antifungal agents, the growth factors and the solution of Tetrazolium must be sterilised by a 22µm filtration. The medium in then distributed in sterile Petri dishes, dried and kept at a temperature of 4 - 7°C, until used.

When the specimen has been seminated and a certain period of incubation, that must be done at 37°C in an atmosphere containing approximately 5% oxygen, 10% carbon dioxide and 85% nitrogen, has passed, the differentiation of the Helicobacter pylori is carried out directly on the primary culture of the Petri dish via simple observation of the characteristics and of the colour of the bacteria colonies grown on it.

### Example:

The reading of the plates is done after 48 - 72 hours of culture for the primary cultures and after 24 - 48 hours for the subcultures. The colonies of Helicobacter pylori will have a red colour with golden reflections because of the reduction of the Tetrazolium salts, a bright appearance and dimensions comprised between 0,5 and 3 mm depending on the incubation time. Furthermore, with the aforementioned medium, cultures of Helicobacter pylori strains, that have taken the typical coccoidal conformation and are not cultivable with common culture media used for the growth of this germ, may be prepared.

## Claims

1. Culture medium for isolating and typing helicobacteria, characterised in that it comprises a suspension of egg yolk or its components.

2. Culture medium according to claim 1, wherein said suspension is obtained from fresh chicken or duck's eggs.

3. Culture medium according to claim 1, or 2, wherein the quantity of said suspension is between 2 ml and 200 ml per 1000 ml of culture medium.

4. Culture medium according to claim 3, wherein the quantity of said suspension is 30 ml per 1000 ml of culture medium.

5. Culture medium according to any preceding claim, further comprising at least one component chosen in a group comprising:
- a nutritive base suitable for the growth of the helicobacter pylori;
- a differentiating substrate comprising tetrazolium chloride;
- a mixture of growth factors;
- a gel substance comprising agar in a water solution.

6. Culture medium according to claim 5, further comprising a mixture of selective substances that inhibits the growth of fungi and Gram positive and Gram negative microorganisms different from the helicobacteria to be selected.

7. Culture medium according to claim 5, or 6, wherein said nutritive base comprises Columbia broth from 5 to 80 g per litre of culture medium.

8. Culture medium according to claim 7, wherein said nutritive base includes Columbia broth in the quantity of 42,5 g per litre of culture medium.

9. Culture medium according to any preceding claim, wherein said differentiating substrate comprises Tetrazolium chloride from 5 to 150 mg per litre of culture medium.

10. Culture medium according to any one of claims 6 to 9, wherein said mixture of selective substances comprises at least one of the following components:
- Vancomycin from 3,00 to 15,00 mg per litre of culture medium;
- Trimethoprim lactate from 1,50 to 10,00 mg per litre of culture medium;
- Cefsulodin from 1,50 to 10,00 mg per litre of culture medium;
- Colystin from 2,00 to 20,00 mg per litre of culture medium;
- Amphoterycin B from 1,50 to 10,00 mg per litre of culture medium;
- Nistatin from 3000 to 20000 U.I. per litre of culture medium.

11. Culture medium according to claim 10, wherein said mixture of selective substances comprises at least one of the following components:
- Vancomycin 9.80 mg per litre of culture medium;
- Trimethoprim lactate 4,80 mg per litre of culture medium;
- Cefsulodin from 4,80 mg per litre of culture medium;
- Colystin from 7,30 mg per litre of culture medium;
- Amphoterycin B from 4,80 mg per litre of culture medium;
- Nistatin from 12000 U.I. per litre of culture medium.

12. Culture medium according to any preceding claim, wherein said mixture of growth factors comprises at least one of the following components:
- Dextrose from 200,00 to 5000,00 mg per litre of culture medium;
- Vitamin B12 from 0,02 to 5,00 mg per litre of culture medium;
- L-glutamine from 20,00 to 1000,00 mg per litre of culture medium;
- Cysteine Hcl from 20,00 to 500,00 mg per litre of culture medium;
- L-Cysteine from 2,00 to 50,00 mg per litre of culture medium;
- Thiamine Hcl from 0,01 to 0,20 mg per litre of culture medium;
- Guanine Hcl from 0,05 to 1,00 mg per litre of culture medium;
- Adenine SO₄ from 1,00 to 50,00 mg per litre of culture medium;
- P-amino benzoic acid from 0,05 to 1,00 mg per litre of culture medium;
- Cocarboxylase from 0,023 to 10,00 mg per litre of culture medium;
- Ferric nitrate from 0,05 to 2,00 mg per litre of culture medium.

13. Culture medium according to claim 12, wherein said mixture of growth factors comprises at least one of the following components:
- Dextrose from 2000,00 mg per litre of culture medium;
- Vitamin B12 from 0,20 mg per litre of culture medium;
- L-glutamine from 200 mg per litre of culture medium;
- Cysteine Hcl from 120 mg per litre of culture medium;
- L-Cysteine from 20 mg per litre of culture medium;
- Thiamine Hcl from 0,06 mg per litre of culture medium;
- Guanine Hcl from 0,60 mg per litre of culture medium;
- Adenine SO₄ from 20,00 mg per litre of culture medium;
- P-amino benzoic acid from 0,26 mg per litre of culture medium;
- Cocarboxylase from 2,00 mg per litre of culture medium;
- Ferric nitrate from 0,40 mg per litre of culture medium.

14. Culture medium according to any preceding claim, wherein said gel substance comprises agar in a water solution from 10 to 20 g per litre of culture medium.

15. Use of a culture medium according to any preceding claim for the isolation and the culture of Helicobacter pylori from biopsy specimens of tissues, from saliva and from stools.

16. Use of a culture medium according to any one of claims 1 to 5, or 7 to 14, when appended to claims 1 to 5, wherein said culture medium is used for the antibiogram of helicobacteria.

17. Use of a culture medium according to claim 16 for the antibiogram of Helicobacter pylori.
